(19) Europäisches Patentamt
European Patent Office
Office européen des brevets

(11) EP 2 750 658 B1

(12) **FASCICULE DE BREVET EUROPEEN**

(45) Date de publication et mention de la délivrance du brevet:
**06.04.2016 Bulletin 2016/14**

(21) Numéro de dépôt: **12756790.7**

(22) Date de dépôt: **24.08.2012**

(51) Int Cl.:
***A61K 8/81*** *(2006.01)* ***A61K 8/365*** *(2006.01)*
***A61K 8/04*** *(2006.01)* ***A61Q 19/10*** *(2006.01)*
***A61Q 19/02*** *(2006.01)* ***A61Q 5/02*** *(2006.01)*
***A61Q 1/14*** *(2006.01)*

(86) Numéro de dépôt international:
**PCT/FR2012/051931**

(87) Numéro de publication internationale:
**WO 2013/030499 (07.03.2013 Gazette 2013/10)**

# (54) NOUVEAU PROCÉDÉ D'AMÉLIORATION DES PROPRIÉTÉS MOUSSANTES DE FORMULATIONS NETTOYANTES À USAGE TOPIQUE

NEUES VERFAHREN ZUR VERBESSERUNG DER SCHAUMEIGENSCHAFTEN VON REINIGUNGSZUSAMMENSETZUNGEN ZUR TOPISCHEN ANWENDUNG

NOVEL METHOD FOR IMPROVING THE FOAMING PROPERTIES OF CLEANING COMPOSITIONS FOR TOPICAL USE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **31.08.2011 FR 1157699**

(43) Date de publication de la demande:
**09.07.2014 Bulletin 2014/28**

(73) Titulaire: **Societe D Exploitation De Produits Pour Les Industries Chimiques Seppic**
**75007 Paris (FR)**

(72) Inventeur: **MERAT, Emmanuelle**
**F-81440 Lautrec (FR)**

(74) Mandataire: **Conan, Philippe Claude**
**L'Air Liquide SA**
**Direction de la Propriété Intellectuelle**
**75, quai d'Orsay**
**75321 Paris Cedex 07 (FR)**

(56) Documents cités:
**WO-A1-2011/030044 WO-A2-2008/087326**
**US-A1- 2004 167 055 US-B2- 7 025 973**

- **Mattei A.: "EU07165 GEL SCRUB MASK", SEPPIC , 4 août 2011 (2011-08-04), XP055024728, Extrait de l'Internet: URL:http://www.seppic.com/file/galleryelement/pj/db/2e/8f/c5/eu07165ang%20-%20gel%20scrub%20mask1855302192658020857.pdf [extrait le 2012-04-17]**
- **P. BERNARD ET AL: "A new polymer with a maximum resistance to electrolytes", SÖFW-JOURNAL, vol. 136, no. 12, 1 décembre 2010 (2010-12-01), pages 55-58, XP055024800,**
- **Bernard P.: "Seppic's polymer with a maximum resistance to electrolytes", LICA Enterprises Group , 1 mars 2010 (2010-03-01), XP002674160, Extrait de l'Internet: URL:http://lica.com.tw/english/licadata/seppic/SepiMAX%20ZEN [extrait le 2012-04-18]**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention se rapporte au domaine de l'industrie cosmétique et/ou pharmaceutique. Elle a pour objet un nouveau procédé pour améliorer les propriétés moussantes de formulations nettoyantes et/ou moussantes, de nouvelles compositions et leurs procédés de préparation.

Les formules nettoyantes pour le visage, pour le corps et pour les cheveux, et plus généralement les produits d'hygiène corporelle et capillaire présentées sous forme de shampoings, de lotions, de gels ou de savons liquides, requièrent la formation de mousse lors de leur application sur la partie du corps à nettoyer. En effet, dans l'esprit du consommateur la formation de mousse constitue une des preuves de l'efficacité du nettoyage. Le volume de cette mousse, sa stabilité, ainsi que les sensations agréables qu'elle induit, sont des paramètres importants à prendre en compte pour espérer le succès commercial de ces formulations.

Pour se faire, ces formutations nettoyantes comprennent des tensioactifs nettoyants et moussants, qu'ils soient de nature cationique, anionique, amphotère ou non ionique.

Comme la surface de la peau présente une valeur de pH faiblement acide, généralement comprise entre 4,0 et 6,5 (à l'exception des peaux grasses qui présentent une valeur de pH supérieure à 6,5), les formulations nettoyantes doivent avoir un pH du même ordre pour ne pas bouleverser le pH de la peau et lui conférer un caractère gras.

Les formulations nettoyantes du visage, du corps et des cheveux, doivent avoir une consistance suffisante pour que leur application sur la surface de la peau à nettoyer soit efficace. C'est pourquoi elles comprennent souvent des agents épaississants compatibles avec les tensioactifs nettoyants et moussants, qui leur donnent cette consistance.

Parmi les épaississants couramment utilisés pour épaissir ces formulations, il y a le chlorure de sodium ; à une dose optimale, il induit la formation de micelles de tensioactifs, qui diminuent les mouvements du fluide et en augmente alors la viscosité. Ce moyen d'épaississsement est peu onéreux. Cependant, la mousse lors du lavage est peu stable, la taille des bulles d'air la composant est importante, ce qui a pour conséquence lors du nettoyage du visage d'augmenter le risque contact avec les yeux et donc d'irritation oculaire, se traduisant par un picotement des yeux inconfortable pour le consommateur.

Les hydrocolloïdes d'origine végétale ou biosynthétique, comme par exemple la gomme de xanthane, la gomme de karaya, les carraghénates, les alginates, les galactomannanes, sont également des agents épaississants couramment utilisés dans l'industrie cosmétique, car l'épaississement qu'ils induisent est peu sensible à la présence des espèces électrolytiques contenues dans les formulations de nettoyage de la peau. La texture de ces formulations est cependant « filante », en ce qu'elles adhèrent trop aux surfaces dures et à la peau, ce qui est un inconvénient, à la fois sur les chaînes de conditionnement car elles entraînent une augmentation du temps de lavage du matériel mis en oeuvre, et lors de la préhension par le consommateur avant l'application sur la peau.

Les copolymères anioniques réticulés à base d'acide méthacrylique ou d'acide acrylique, ou d'esters de l'acide méthacrylique ou de l'acide acrylique, optionnellement hydrophiquement modifiés, préparés par polymérisation en émulsion directe sont également utilisés en cosmétique. Ils sont respectivement connus de l'homme du métier sous les appellations "Alcaline Swellable Emulsion" (ou "ASE") et "Hydrophobically Alcaline Swellable Emulsion" (ou "HASE"). Des agents épaississants de type « "HASE" » sont décrits dans la demande internationale publiée le 2 mai 2002 sous le numéro WO2002/34793 A2. Leur efficacité d'épaississement n'est cependant satisfaisante qu'à partir d'un pH supérieur ou égal à 6,5. La texture des formulations est de type "gélifiée", ce qui dans ce cas aussi est un inconvénient lors de la préhension par le consommateur avant application sur la peau.

Il est aussi connu de l'homme du métier d'utiliser des polyélectrolytes anioniques réticulés ou branchés, qui sont des homopolymères ou des copolymères réticulés et/ou branchés de monomères insaturés hydrosolubles, comme par exemple l'acide acrylique ou les dérivés de l'acide acrylique, l'acrylamide, ou les dérivés de l'acrylamide, l'acide acrylamidométhyl propanesulfonique commercialisés sous les noms CARBOPOL™, ULTREZ™ 10, PEMULEN™ TR1, PEMULEN™ TR2, SIMULGEL™ EG, SIMULGEL™ EPG, LUVIGEL™ EM, SALCARE™ SC91, SALCARE™ SC92, SALCARE™ SC95, SALCARE™ SC96, FLOCARE™ ET100, FLOCARE™ ET58, HISPAGEL™, SEPIGEL™ 305, SEPIGEL™ 501, SEPIGEL™ 502, SIMULGEL™ NS, SIMULGEL™ 800, SIMULGEL™600, SIMULGEL™ A, SEPIPLUS™ 250, SEPIPLUS™ 265, SEPIPLUS™ 400, SEPINOV™ EMT 10, NOVEMER™ EC1, ARISTOFLEX™ AVC, ARISTOFLEX™ HBM, RAPITHIX™ A60, RAPITHIX™ A100, COSMEDIA SP et STABILEZE™ 06. Ces polyélectrolytes anionique réticulés ou branchés se présentent sous la forme de latex inverses, obtenus par polymérisation radicalaire en émulsion inverse, ou sous la forme de poudres, obtenues par polymérisation précipitante ou par atomisation de latex inverses. Leur utilisation comme agent épaississant ne s'avère toutefois pas suffisamment efficace pour épaissir en présence des espèces électrolytiques contenus dans les formulations de nettoyage de la peau.

Les polyélectrolytes anioniques réticulés ou branchés, comme ceux décrits dans la demande de brevet internationale publiée sous le numéro WO 2011/030044, épaississent efficacement les formulations de nettoyage de la peau, mais leur utilisation à une valeur de pH supérieure ou égale à 7,0 en présence de tensioactifs moussants ne permet cependant pas d'obtenir une mousse suffisamment fine.

Le brevet américain US 7,025,973 B2 décrit des copolymères obtenus par polymérisation radicalaire de l'acide

2-méthyl 2-[(1-oxo 2-propènyl) amino] 1-propanesulfonique avec des monomères oléfiniques insaturés, comprenant au moins un atome d'oxygène ou d'azote, de poids moléculaire inférieur à 500 g/mol, et au moins un macromère. Le brevet américain US 7,025,973 B2 ne divulgue pas de compositions cosmétiques comprenant des polyélectrolytes anioniques réticulés ou branchés, tels que décrits dans la demande internationale publiée sous le numéro WO 2011/030044, et des tensioactifs moussants, ni l'utilisation desdits polyélectrolytes anioniques réticulés ou branchés pour préparer des compositions cosmétiques nettoyantes générant un faible volume de mousse, de façon à limiter le risque du contact de ladite mousse avec les yeux.

Or, dans le cas particulier du nettoyage du visage, qui vise à désincruster les pores de la peau, ou à éliminer la peau des impuretés, de l'excès de sébum, des cellules mortes, ou des traces de maquillage, il est préférable que les formulations nettoyantes génèrent un faible volume de mousse et que les bulles d'air qui composent cette mousse soient stables et soient une taille moyenne suffisament fine pendant la phase de nettoyage, de façon à limiter le risque contact avec les yeux et donc d'irritation oculaire se traduisant par un picotement des yeux inconfortable pour le consommateur.

Pour tenter de s'affranchir de ces inconvénients, les inventeurs ont donc cherché à développer une nouvelle solution permettant de disposer d'une formulation de nettoyage qui présente une valeur de pH supérieure ou égale à 4,0 et inférieure ou égale à 6,5, plus particulièrement supérieure ou égale à 6,0 et inférieure ou égale à 4,5, qui soit facile à la préhension par le consommateur, et qui génère une mousse fine et stable lors de sa mise en oeuvre dans l'opération de nettoyage de la peau, et plus particulièrement du visage.

C'est pourquoi selon un premier aspect, l'invention a pour objet un procédé pour améliorer les propriétés moussantes d'une formulation nettoyante à usage topique, de pH supérieur ou égal à 4,0 et inférieur ou égale à 6,5 et comprenant au moins un agent tensioactif moussant, ledit procédé étant caractérisé en ce que l'on incorpore dans ladite formulation nettoyante à usage topique, une quantité efficace d'un polyélectrolyte anionique réticulé (P) issu de la polymérisation, en présence d'au moins un agent de réticulation, d'au moins un monomère possédant une fonction acide fort, ledit monomère étant l'acide 2-méthyl 2-[(1-oxo 2-propènyl) amino] 1-propanesulfonique, partiellement ou totalement salifiée, avec au moins un monomère neutre choisi parmi les N,N-dialkyl acrylamides, dans lesquels chacun des groupes alkyle comportent entre un et quatre atomes de carbones,et au moins un monomère de formule (I) :

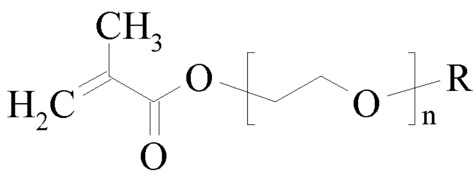

(I)

dans laquelle R représente un radical alkyle linéaire ou ramifié comportant de huit à vingt atomes de carbone et n représente un nombre supérieur ou égal à un et inférieur ou égal à vingt.

Par agent tensioactif moussant, on désigne, dans la définition du procédé tel que défini ci-dessus, tout agent tensioactif moussant, qu'il soit anionique, cationique, amphotère ou non ionique, topiquement acceptable.

Par quantité efficace d'un polyélectrolyte anionique réticulé (P), on désigne une quantité telle que :

- Le diamètre moyen d'au moins 70% des bulles d'air présentes dans la mousse formée par la formulation nettoyante, soit inférieur ou égal à 150 micromètres (μm) après une durée de 10 minutes à compter du moment de la génération de ladite mousse ;
- Le diamètre moyen d'au plus 30% des bulles d'air présentes dans la mousse formée par la formulation nettoyante soit supérieur ou égal à 150 μm et inférieur ou égal à 450 μm après une durée de 10 minutes à compter du moment de la génération de ladite mousse ;
- Et telle que la viscosité de la formulation nettoyante préparée soit supérieure ou égale à 2 000 mPa.s et inférieure ou égale à 30 000 mPa.s, plus particulièrement supérieure ou égale à 2 000 mPa.s et inférieure ou égale à 20 000 mPa.s, mesurée à une température de 20°C au moyen d'un viscosimètre de type Brookfield LVT à une vitesse de 6 tours/minutes.

L'expression "à usage topique" utilisée dans la définition du procédé tel que défini ci-dessus, signifie que ladite formulation nettoyante à usage topique est mise en oeuvre par application sur la peau du corps et du visage, sur les cheveux, sur le cuir chevelu ou sur les muqueuses, qu'il s'agisse d'une application directe dans le cas d'une formulation cosmétique, dermocosmétique, dermo-pharmaceutique ou pharmaceutique ou d'une application indirecte par exemple dans le cas d'un produit d'hygiène corporelle sous forme de lingette en textile ou en papier ou de produits sanitaires destinés à être en contact avec la peau ou les muqueuses.

Par polyélectrolyte anionique réticulé, on désigne, dans la définition du procédé tel que défini ci-dessus, un

polyélectrolyte anionique réticulé non linéaire se présentant à l'état de réseau tridimensionnel insoluble dans l'eau, mais gonflable à l'eau et conduisant donc à l'obtention d'un gel chimique.

Par partiellement salifié ou totalement salifié, on signifie, dans la définition du procédé tel que défini ci-dessus, que ladite fonction acide fort du monomère en comportant est, partiellement ou totalement salifiée, généralement sous forme de sel de métal alcalin, comme par exemple le sel de sodium ou le sel de potassium, ou sous forme de sel d'ammonium.

Dans le cadre du procédé tel que défini ci-dessus et objet de la présente invention, ledit polyélectrolyte anionique réticulé (P) mis en oeuvre comporte généralement entre 5% molaire et 95% molaire du monomère à fonction acide fort, plus particulièrement entre 10% molaire et 90% molaire, notamment entre 20% molaire et 80% molaire, et tout particulièrement entre 60% molaire et 80% molaire.

Dans le cadre du procédé objet de la présente invention, ledit polyélectrolyte anionique réticulé (P) tel que défini précédemment, comporte plus particulièrement entre 4,9% molaire et 90% molaire de monomère neutre choisi parmi les N,N-dialkyl acrylamides, dans lesquels chacun des groupes alkyle comportent entre un et quatre atomes de carbones, plus particulièrement entre 9,5% molaire et 85% molaire, notamment entre 15% molaire et 75% molaire, et tout particulièrement entre 15% molaire et 39,5% molaire.

Dans ledit polyélectrolyte anionique réticulé (P) mis en oeuvre dans le procédé objet de la présente invention et tel que défini ci-dessus, la fonction acide fort du monomère en comportant est notamment la fonction acide sulfonique, partiellement ou totalement salifiée.

Selon un aspect particulier du procédé tel que défini ci-dessus, par quantité efficace d'un polyélectrolyte anionique réticulé (P), on désigne, pour 100% massique de la formulation nettoyante, une proportion massique endit polyélectrolyte anionique (P), comprise entre 0,1% et 2,0%, plus particulièrement entre 0,1% massique et 1,5% massique, et encore plus particulièrement entre 0,1% massique et 1% massique.

Le monomère neutre choisi parmi les N,N-dialkyl acrylamides, dans lesquels chacun des groupes alkyle comportent entre un et quatre atomes de carbones, est notamment choisi parmi, le N,N-diméthyl acrylamide, le N,N-diéthyl acrylamide, le N,N-dipropyl acrylamide.

Dans le cadre de la présente invention, ledit polyélectrolyte anionique réticulé (P) tel que défini précédemment, comporte entre 0,1% molaire et 10% molaire de monomères de formule (I) et plus particulièrement entre 0,5% molaire et 5% molaire de monomères de formule (I).

Dans la formule (I) du monomère présent dans ledit polyélectrolyte anionique réticulé (P) mis en oeuvre dans le procédé objet de la présente invention, par radical alkyle linéaire ou ramifié comportant de huit à vingt atomes de carbone, on désigne plus particulièrement pour R :

- <u>ou bien</u> un radical dérivé des alcools primaires linéaires tels que par exemple, le radical octyle, décyle, undécyle, dodécyle, tridécyle, tétradécyle, pentadécyle, hexadécyle, heptadécyle, octadécyle, nonadécyle ou eicosyle ;
- <u>ou bien</u> un radical dérivé des alcools de Guerbet, qui sont des 1-alcanols ramifiés répondant à la formule générale :

  $$CH_3\text{-}(CH2)_p\text{-}CH[CH_3\text{-}(CH_2)_{p\text{-}2}]\text{-}CH_2OH,$$

  dans laquelle p représente un nombre entier compris entre 2 et 9, tels que, par exemple, les radicaux 2-éthyl hexyle, 2-propyl heptyle, 2-butyl octyle, 2-pentyl nonyle, 2-hexyl décyle ou 2-octyl dodécyle ;
- <u>ou bien</u> un radical dérivé des isoalcanols répondant à la formule générale :

  $$CH_3\text{-}CH(CH_3)\text{-}(CH_2)_m\text{-}CH_2OH,$$

dans laquelle m représente un nombre entier compris entre 2 et 16, tels que, par exemple, les radicaux 4-méthyl pentyle, 5-méthyl hexyle, 6-méthyl heptyle, 15-méthyl pendadécyle ou 16-méthyl heptadécyle, <u>soit</u> les radicaux 2-hexyl octyle, 2-octyl décyle ou 2-hexyl dodécyle.

Selon un aspect particulier, l'invention a pour objet un procédé tel que décrit précédemment, caractérisé en ce que ledit polyélectrolyte anionique réticulé (P) comporte pour 100% molaire :

- De 20% molaire à 80% molaire d'unités monomériques issues du monomère comportant une fonction acide fort partiellement ou totalement salifiée ;
- De 15% molaire à 75% molaire d'unités monomériques issues d'un monomère neutre choisi parmi les N,N-dialkyl acrylamides, dans lesquels chacun des groupes alkyle comportent entre un et quatre atomes de carbones ;
- De 0,5% à 5% molaire d'unités monomériques issues d'un monomère de formule (I) telle que définie précédemment.

Selon un autre aspect particulier de la présente invention, celle-ci à pour objet un procédé tel que précédemment,

caractérisé en ce que dans ledit polyélectrolyte anionique réticulé (P), ledit monomère possédant une fonction acide fort est l'acide 2-méthyl 2-[(1-oxo 2-propènyl) amino] 1-propanesulfonique partiellement salifié ou totalement salifié et plus particulièrement l'acide 2-méthyl 2-[(1-oxo 2-propènyl) amino] 1-propanesulfonique partiellement salifié ou totalement salifié sous forme de sel de métal alcalin, comme par exemple le sel de sodium ou le sel de potassium, ou sous forme de sel d'ammonium.

Selon un aspect particulier de la présente invention, celle-ci a pour objet le procédé tel que défini précédemment, caractérisé en ce que dans ledit polyélectrolyte anionique réticulé (P), ledit monomère neutre est le N,N-diméthyl acrylamide.

Selon un aspect particulier de la présente invention, celle-ci a pour objet le procédé tel que défini précédemment, caractérisé en ce que dans ledit polyélectrolyte anionique réticulé (P) et pour ledit monomère de formule (I) telle que définie précédemment, R représente un radical alkyle comportant de 12 à 18 atomes de carbone.

Selon un autre aspect particulier, l'invention a pour objet le procédé tel que défini précédemment, caractérisé en ce que dans ledit polyélectrolyte anionique réticulé (P) et pour ledit monomère de formule (I) telle que définie précédemment, n représente un nombre entier compris entre 3 et 20.

Selon un aspect encore plus particulier, l'invention a pour objet le procédé tel que défini précédemment, caractérisé en ce que dans ledit polyélectrolyte anionique réticulé (P), ledit monomère de formule (I) est le méthacrylate de lauryle tétraéthoxylé.

Selon un aspect encore plus particulier, l'invention a pour objet le procédé tel que défini précédemment pour lequel le monomère de formule (I) compris dans le polyélectrolyte anionique réticulé (P) est le méthacrylate de stéaryle eicosaéthoxylé.

Selon un autre aspect particulier, l'invention a pour objet le procédé tel que défini précédemment pour lequel ledit polyélectrolyte anionique réticulé (P) est réticulé avec un composé diéthylénique ou polyéthylènique dans la proportion molaire exprimée par rapport aux monomères mis en oeuvre, de 0,005% à 1%, plus particulièrement de 0,01% à 0,5% et tout particulièrement de 0,01% à 0,25%. L'agent de réticulation est plus particulièrement choisi parmi le diméthacrylate d'éthylèneglycol, le tétraallyloxyéthane, le diacrylate d'éthylèneglycol, le diallyl urée, le triallyl amine, le triméthylol propanetriacrylate ou le méthylène-bis(acrylamide) ou un mélange de ces composés.

Le polyélectrolyte anionique réticulé (P) mis en oeuvre dans le procédé objet de la présente invention peut également comprendre divers additifs, tels que des agents complexants, des agents de transfert ou des agents limiteurs de chaîne.

Selon un aspect particulier, l'invention a pour objet un procédé tel que décrit précédemment pour lequel ledit polyélectrolyte anionique réticulé (P) est choisi parmi les terpolymères de l'acide 2-méthyl 2-[(1-oxo 2-propènyl) amino] 1-propanesulfonique partiellement salifié sous forme de d'ammonium, du N,N-diméthyl acrylamide et du méthacrylate de lauryle tétraéthoxylé, réticulé au triméthylol propanetriacrylate ou les terpolymères de l'acide 2-méthyl 2-[(1-oxo 2-propènyl) amino] 1-propanesulfonique partiellement salifié sous forme de sel d'ammonium, du N,N-diméthyl acrylamide et du méthacrylate de stéaryle d'eicosaéthoxylé, réticulé au triméthylol propanetriacrylate.

Selon un aspect encore plus particulier, l'invention a pour objet un procédé tel que décrit précédemment pour lequel ledit polyélectrolyte anionique réticulé (P) est un terpolymère de l'acide 2-méthyl 2-[(1-oxo 2-propènyl) amino] 1-propanesulfonique partiellement salifié sous forme d'ammonium, du N,N-diméthyl acrylamide et du méthacrylate de lauryle tétraéthoxylé, réticulé au triméthylol propanetriacrylate.

Selon un aspect encore plus particulier, l'invention a pour objet un procédé tel que décrit précédemment pour lequel ledit polyélectrolyte anionique réticulé (P) comporte pour 100% molaire :

- de 60% molaire à 80% molaire d'unités monomériques issues de l'acide 2-méthyl 2-[(1-oxo 2-propènyl) amino] 1-propanesulfonique partiellement salifié sous forme d'ammonium,
- de 15% molaire à 39,5% molaire d'unités monomériques issues du N,N-diméthyl acrylamide, et
- de 0,5% molaire à 5% molaire d'unités monomériques issues du méthacrylate de lauryle tétraéthoxylé

Dans le procédé objet de la présente invention et tel que défini ci-dessus, parmi les tensioactifs anioniques que l'on peut associer au polyélectrolyte anionique réticulé (P) dans les formulations nettoyantes à usage topique et présentant une valeur de pH supérieure ou égale à 4,0 et inférieure ou égale à 6,5, on citera particulièrement les sels de métaux alcalins, les sels de métaux alcalino-terreux, les sels d'ammonium, les sels d'amines, les sels d'aminoalcools des composés suivants : les alkyléthers sulfates, les alkylsulfates, les alkylamidoéthersulfates, les alkylarylpolyéthersulfates, les monoglycérides sulfates, les alpha-oléfinesulfonates, les paraffines sulfonates, les alkylphosphates, les alkylétherphosphates, les alkylsulfonates, les alkylamidesulfonates, les alkylarylsulfonates, les alkylcarboxylates, les alkylsulfosuccinates, les alkyléthersulfosuccinates, les alkylamidesulfosuccinates, les alkylsulfoacétates, les alkylsarcosinates, les acyliséthionates, les N-acyltaurates, les acyllactylates. Parmi les tensioactifs anioniques, on citera également les lipoaminoacides, les lipoprotéines, les lipopeptides, les dérivés des lipoprotéines, les dérivés de protéines, les sels d'acides gras, les sels d'acides d'huile de coprah éventuellement hydrogénée.

Selon un aspect plus particulier de la présente invention, celle-ci a pour objet un procédé tel que défini précédemment, pour lequel la formulation nettoyante à usage topique comporte au moins un agent tensioactif moussant anionique de formule (II) :

$$R_2\text{-O-}(CH_2\text{-}CH_2\text{-O})_pSO_3\text{-X} \qquad (II)$$

dans laquelle $R_2$ représente un radical hydrocarboné aliphatique, saturé ou insaturé, linéaire ou ramifié, comportant de 6 à 22 atomes de carbone, p représente un nombre décimal compris entre 1 et 10, de préférence entre 2 et 4, et X représente le cation d'un métal alcalin ou d'un métal alcalino-terreux, l'ion ammonium, l'ion hydroxyéthyl ammonium, l'ion tris(hydroxyéthyl) ammonium, ou un mélange de composés de formule (II).

Dans la formule (II) telle que définie ci-dessus, X représente par exemple le sodium, le magnésium ou le l'ion ammonium.

Dans le procédé objet de la présente invention et tel que défini ci-dessus, parmi les tensioactifs amphotères que l'on peut associer au polyélectrolyte anionique réticulé (P) dans les formulations nettoyantes à usage topique et présentant une valeur de pH supérieure ou égale à 4,0 et inférieure ou égale à 6,5, on citera particulièrement les alkylbétaines, les alkylamidobétaines, les sultaines, les alkylamidoalkylsulfobétaines, les dérivés d'imidazolines, les phosphobétaïnes, les amphopolyacétates et les amphopropionates.

Selon un aspect plus particulier de la présente invention, celle-ci a pour objet un procédé tel que défini précédemment pour lequel la formulation nettoyante à usage topique comporte au moins un agent tensioactif moussant amphotère de formule (III) :

$$R_3\text{-C(O)-NH}(CH_2)q\text{-}N^+(R_4)(R_5)\text{-}(CH_2)_s\text{-}CO_2^- \qquad (III)$$

dans laquelle $R_3$ représente un radical hydrocarboné aliphatique, saturé ou insaturé, linéaire ou ramifié, comportant de 7 à 21 atomes de carbone, $R_4$ et $R_5$ représentent indépendamment l'un de l'autre un radical aliphatique, saturé ou insaturé, linéaire ou ramifié, éventuellement substitué avec un groupe hydroxyle, comportant de 1 à 4 atomes de carbone, q représente un nombre entier compris entre 2 et 6, et s représente un nombre entier égal à 1 ou à 2, ou un mélange de composés de formule (III).

Dans la formule (III) telle que définie ci-dessus, $R_3$-C(O)- représente par exemple le radical octanoyle, le radical décanoyle, le radical lauroyle, le radical cocoyle.

Dans la formule (III) telle que définie ci-dessus, q est par exemple égal à 3.

Dans la formule (III) telle que définie ci-dessus, $R_4$ et $R_5$ représentent un radical méthyl.

Selon un aspect plus particulier de la présente invention, celle-ci a pour objet un procédé tel que défini précédemment pour lequel l'agent tensioactif moussant amphotère de formule (III) est la cocamidopropryl bétaïne.

Selon un autre aspect particulier de la présente invention, celle-ci a pour objet un procédé tel que défini précédemment, pour lequel la formulation nettoyante à usage topique comporte un mélange d'au moins un composé de formule (II), telle que définie précédemment, avec au moins un composé de formule (III) telle que définie précédemment.

Dans le procédé objet de la présente invention et tel que défini ci-dessus, parmi les tensioactifs non ioniques que l'on peut associer au polyélectrolyte anionique réticulé (P) dans les formulations nettoyantes à usage topique et présentant une valeur de pH supérieure ou égale à 4,0 et inférieure ou égale à 6,5, on citera particulièrement les dérivés éthoxylés d'alcools gras comportant de 8 à 16 atomes de carbone, les dérivés éthoxylés d'acides gras comportant de 8 à 16 atomes de carbone, les dérivés éthoxylés d'esters gras comportant de 8 à 16 atomes de carbone, les dérivés éthoxylés de monoglycérides comportant de 8 à 16 atomes de carbone, les dérivés éthoxylés de sorbitan, les dérivés éthoxylés de mannitan, les alkylpolyglycosides, les dérivés d'huile de ricin, les polysorbates, les amides de coprah, les N-alkylamines, les oxydes d'amines.

Parmi les tensioactifs non ioniques moussants cités ci-dessus, qui sont des tensioactifs non-ioniques, il y a plus particulièrement les composés de formule (IV) :

$$R_6\text{-O-}(S)_y\text{-H} \qquad (IV)$$

dans laquelle y représente un nombre décimal compris entre 1 et 5, S représente le reste d'un sucre réducteur et $R_6$ représente un radical alkyle linéaire ou ramifié, saturé ou insaturé, ayant de 8 à 16 atomes de carbone, de préférence de 8 à 14 atomes de carbone, ou un mélange de composés de formule (IV).

Dans la définition de la formule (IV) telle que définie précédemment, y est un nombre décimal qui représente le degré moyen de polymérisation du reste S. Lorsque y est un nombre entier, $(S)_y$ est le reste polymérique de rang y du reste S. Lorsqu'y est un nombre décimal, la formule (IV) représente un mélange de composés :

$a_1 R_6$-O-S-H + $a_2 R_6$-O-$(S)_2$-H + $a_3 R_6$-O-$(S)_3$-H + ... + $a_q R_6$-O-$(S)_q$-H avec q représentant un nombre entier compris entre 1 et 10 et dans les proportions molaires $a_1$, $a_2$, $a_3$,... $a_q$ telles que :

$$\sum_{q=10}^{q=1} a_q = 1 \; ; \; a_1 > 0$$

Dans le procédé objet de la présente invention et tel que défini ci-dessus, dans la définition des composés de formules (IV), y est compris entre 1,05 et 5, et plus particulièrement entre 1,05 et 2.

Dans la formule (IV) telle que définie ci-dessus, $R_6$ représente par exemple le radical n-octyle, le radical n-décyle, le radical n-dodécyle, le radical n-dodécyle ou le radical n-tétradécyle.

Par sucre réducteur, on désigne, dans la définition de la formule (IV), les dérivés saccharidiques qui ne présentent pas dans leurs structures de liaison glycosidique établie entre un carbone anomérique et l'oxygène d'un groupement acétal tels qu'ils sont définis dans l'ouvrage de référence « Biochemistry », Daniel Voet/Judith G. Voet, p. 250, John Wyley & Sons, 1990. La structure oligomérique $(S)_y$, peut se présenter sous toute forme d'isomérie, qu'il s'agisse d'isomérie optique, d'isomérie géométrique ou d'isomérie de position ; elle peut aussi représenter un mélange d'isomères.

Dans la formule (IV) telle que définie ci-dessus, le groupe $R_6$-O- est lié à S par le carbone anomérique du reste saccharide, de manière à former une fonction acétal.

Selon un aspect plus particulier de la présente invention, celle-ci a pour objet un procédé tel que défini précédemment pour lequel la formulation nettoyante à usage topique comprend au moins un composé de formule (IV) dans la quelle y est un nombre décimal compris entre 1,05 et 2, S représente le reste d'un sucre réducteur choisi parmi le glucose, le xylose ou l'arabinose et $R_6$ représente un radical choisi parmi les radicaux n-octyle, n-décyle, n-dodécyle, n-tétradécyle, n-hexadécyle, au un mélange de composés de formule (IV).

Selon un autre aspect particulier de la présente invention, celle-ci a pour objet un procédé tel que défini précédemment, caractérisé en ce que le rapport massique agent tensioactf moussant sur polyélectrolyte anionique réticulé (P) est compris entre 1/10 et 40/1, plus particulièrement entre 1/1 et 40/1 et encore plus particulièrement entre 4/1 et 40/1.

L'invention a aussi pour objet une composition (C1) caractérisée en ce qu'elle comprend pour 100% de sa masse :

- De 0,05 % à 2 % massique d'au moins un polyélectrolyte anionique réticulé (P) issu de la polymérisation d'au moins un monomère possédant une fonction acide fort, ledit monomère étant l'acide 2-méthyl 2-[(1-oxo 2-propènyl) amino] 1-propanesulfonique, partiellement ou totalement salifiée, avec au moins un monomère neutre choisi parmi les N,N-dialkyl acrylamides, dans lesquels chacun des groupes alkyle comportent entre un et quatre atomes de carbones, et au moins un monomère de formule (I) :

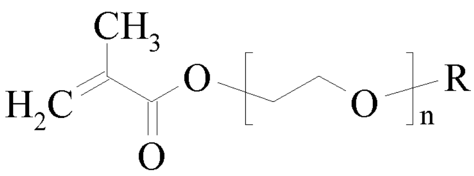

(I)

dans laquelle R représente un radical alkyle linéaire ou ramifié comportant de huit à vingt atomes de carbone et n représente un nombre supérieur ou égal à un et inférieur ou égal à vingt, en présence d'au moins un agent de réticulation ;
- De 10 % à 50% massique d'au moins un tensioactif moussant sélectionné parmi éléments du groupe constitué par les agents tensioactifs anioniques, les agents tensioactifs non ioniques et les agents tensioactifs amphotères.
- de 0,01 % à 10 % massique d'au moins un agent acide (A) sélectionné parmi le groupe constitué par les $\alpha$-hydroxy acides et les $\beta$-hydroxy acides, libres, partiellement ou totalement salifiés.
- de 89,94 % à 38 % massique d'eau,

et en ce que son pH est supérieur ou égal à 4,0 et inférieur ou égal à 6,5.

Selon un aspect particulier, dans la composition (C1) objet de la présente invention, dans le polyélectrolyte (P) tel que défini précédemment, le monomère neutre est choisi parmi le N,N-diméthyl acrylamide, le N,N-diéthyl acrylamide, le N,N-dipropyl acrylamide.

Selon un aspect plus particulier, dans la composition (C1) objet de la présente invention, dans le polyélectrolyte (P) tel que défini précédemment, le monomère neutre est le N,N-diméthyl acrylamide.

Selon un aspect particulier, dans la composition (C1) objet de la présente invention, l'agent tensioactif moussant comprend au moins un composé de formule (II) telle que définie ou au moins un composé de formule (III) telle que définie ci-dessus, ou un mélange d'au moins un composé de formule (II) avec au moins un composé de formule (III).

Selon un autre aspect partculier la composition (C1) telle que définie précédemment, est caractérisée en ce que le rapport massique agent moussant sur polyélectrolyte anionique réticulé (P) est compris entre 1/10 et 40/1, plus particulièrement entre 1/1 et 40/1 et encore plus particulièrement entre 4/1 et 40/1.

Par partiellement ou totalement salifiés, on signifie notamment pour les agents acides (A), partiellement ou totalement salifiés sour forme de sels alcalins, tels que le sel de sodium ou le sel de potassium, de sels alcalino-terreux ou de sel d'ammonium..

Par $\alpha$-hydroxy acide, on désigne notamment, dans le définition de la composition (C1) objet de la présente invention, les composés de formule formule (V) :

$$R_7R_8C(OH)COOH \qquad (V)$$

dans laquelle $R_7$ et $R_8$ représentent indépendamment l'un de l'autre un radical choisi parmi l'atome d'hydrogène, le radical méthyle, le radical -CH(OH)COOH, le radical $-CH_2COOH$, le radical -COOH, le radical $-(CHOH)_k-CH_2OH$ dans lequel k représente un nombre entier compris entre 1 et 6 ou le radical phényle.

Par $\beta$ -hydroxy acide, on désigne notamment, dans le définition de la composition (C1) objet de la présente invention, l'acide 3-hydroxy butanoïque, l'acide 3-hydroxy-3-methylbutanoïque, la carnitine, l'acide salicylique et par les dérivés alcylés de l'acide salicylique de formule (VI) :

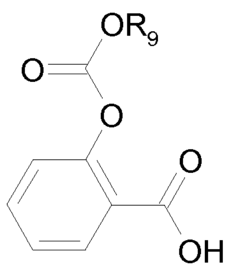

(VI)

dans laquelle $R_9$ représente un radical hydrocarboné aliphatique, saturé ou insaturé, linéaire ou ramifié, comportant de 2 à 22 atomes de carbone, comme par exemple l'acide 2-octanoyloxy-benzoïque, l'acide 2-décanoyloxy benzoïque, l'acide 2-dodécanoyloxy benzoïque, et de leurs sels correspondants.

Selon un aspect particulier, la composition (C1) telle que définie précédemment, est caractérisée en ce que l'agent acide (A) est choisi parmi lactique, l'acide citrique, l'acide glycolique, l'acide gluconique, l'acide tartrique, l'acide malique, l'acide salicylique

La composition (C1) objet de la présente invention se présente notamment sous la forme d'une solution, d'une émulsion ou d'une microémulsion à phase continue aqueuse, d'une émulsion ou d'une microémulsion à phase continue huileuse, d'un gel, d'une mousse, ou encore sous la forme d'un aérosol.

De façon générale la composition (C1) objet de la présente invention comporte, en plus dudit tensioactif moussant dudit agent acide (A) tels que définis précédemment, et de l'eau, des adjuvants et ou des additifs habituellement mis en oeuvre dans le domaine des formulations à usage topique, en particulier cosmétiques, dermocosmétiques, pharmaceutiques ou dermo-pharmaceutiques. Parmi les adjuvants susceptibles d'être présents dans les compositions (C1) objets de la présente invention, on peut citer des agents stabilisants, des composés filmogènes, des solvants et des co-solvants, des agents hydrotropes, des agents plastifiants, des matières grasses, des huiles, des agents émulsionnants et co-émulsionnants, des agents opacificants, des agents nacrants, des agents surgraissants, des agents séquestrants, des agents chélatants, des agents antioxydants, des parfums, des agents conservateurs, des agents conditionneurs, des agents blanchissants destinés à la décoloration des poils et de la peau, des principes actifs destinés à apporter une action traitante vis à vis de la peau ou des cheveux, des charges minérales ou des pigments, des particules procurant un effet visuel ou destinées à l'encapsulation d'actifs, des particules exfoliantes, des agents de texture, des azurants optiques, des repellents pour les insectes.

Parmi les solvants et les co-solvants que peut associer aux compositions (C1) objets de la présente invention, on citera particulièrement des glycols, comme par exemple le butylène glycol, l'hexylène glycol, le caprylyl glycol ou 1,2-octanediol, le pentylene glycol ou 1,2-pentanediol, le pentylène glycol, le monopropylene glycol, le dipropylene glycol, l'isoprène glycol, le butyldiglycol, les polyéthylènes glycols dont le poids moléculaire est compris entre 200 $g.mol^{-1}$

et 8000 g.mol$^{-1}$ ; des alcools comme par exemple l'éthanol, l'isopropanol ; des polyols comme par exemple le glycérol, le diglycérol, le triglycérol, l'érythritol, le xylitol, le sorbitol, le methyl-2,-propanediol-1,3 ; des polyols alcoxylés.

Parmi les émulsionnants que l'on peut associer aux compositions (C1) objets de la présente invention, on citera particulièrement les acides gras comportant de 16 à 22 atomes de carbone, les acides gras éthoxylés comportant de 16 à 22 atomes de carbone, les esters d'acide gras et de sorbitol, les esters de polyglycérol comportant de 16 à 22 atomes de carbone, les alcools gras éthoxylés comportant de 16 à 22 atomes de carbone, les esters de sucrose comportant de 16 à 22 atomes de carbone, les alkylpolyglycosides dont la chaîne alkyle comporte de 16 à 22 atomes de carbone, les alcools gras sulfatés et phosphatés ou les mélanges d'alkylpolyglycosides et d'alcools gras décrits dans les demandes de brevet français 2 668 080, 2 734 496, 2 756 195, 2 762 317, 2 784 680, 2 784 904, 2 791 565, 2 790 977, 2 807 435, 2 804 432, 2 830 774, 2 830 445, les associations de tensioactifs émulsionnants choisis parmi les alkylpolyglycosides, les associations d'alkylpolyglycosides et d'alcools gras, les esters de polyglycérols ou de polyglycols ou de polyols tels que les polyhydroxystéarates de polyglycols ou de polyglycérols mis en oeuvre dans les demandes de brevets français 2 852 257, 2 858 554, 2 820 316 et 2 852 258.

Parmi les agents opacifiants et/ou nacrants que l'on peut associer aux compositions (C1) objets de la présente invention, on citera particulièrement les palmitates ou les stéarates ou les hydroxystéarates de sodium ou de magnésium, les monostéarates ou distéarates d'éthylène ou de polyéthylène glycol, les alcools gras, les homopolymères et copolymères de styrène tels que le styrène acrylate copolymère commercialisé sous l'appellation MONTOPOL™ OP1 par la société SEPPIC.

Comme exemples d'huiles éventuellement présents dans la composition (C1) objet de la présente invention, on peut citer :

- les huiles minérales telles que l'huile de paraffine, l'huile de vaseline, les isoparaffines ou les huiles blanches minérales ;
- les huiles d'origine animale, telles que le squalène ou le squalane,
- les huiles végétales, telles que le phytosqualane, l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula, les huiles issues de fleurs ou de légumes ;
- les huiles végétales éthoxylées ;
- les huiles synthétiques comme les esters d'acides gras tels que le myristate de butyle, le myristate de propyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les huiles hydrogénées, les polyalphaoléfines, les polyoléfines comme le polyisobutène, les isoalcanes de synthèse comme l'isohexadécane, l'isododécane, les huiles perfluorées et
- les huiles de silicone comme les diméthylpolysiloxanes, les méthylphényl - polysiloxanes, les silicones modifiées par des aminés, les silicones modifiés par des acides gras, les silicones modifiés par des alcools, les silicones modifiés par des alcools et des acides gras, des silicones modifiés par des groupements polyéther, des silicones époxy modifiés, des silicones modifiées par des groupements fluorés, des silicones cycliques et des silicones modifiées par des groupements alkyles.

Comme autre matière grasse éventuellement présente dans la formulation pour laquelle on met en oeuvre le procédé objet de la présente invention, on peut citer les alcools gras ou les acides gras ; les cires telles que la cire d'abeille, la cire de carnauba, la cire de candelilla, la cire d'ouricoury, la cire du Japon, la cire de fibre de liège, la cire de canne à sucre, les cires de paraffines, les cires de lignite, les cires microcristallines, la cire de lanoline ; l'ozokérite ; la cire de polyéthylène, les cires de silicone ; les cires végétales ; les alcools gras et les acides gras solides à température ambiante ; les glycérides solides à température ambiante.

Comme exemples de principes actifs autres que les $\alpha$-hydroxy acides et les $\beta$-hydroxy acides présents dans la composition (C1) objet de la présente invention, on peut citer les vitamines et leurs dérivés, notamment leurs esters, tels que le rétinol (vitamine A) et ses esters (palmitate de rétinyle par exemple), l'acide ascorbique (vitamine C) et ses esters (par exemple l'ascorbyl phosphate de magnésium), les dérives de sucre de l'acide ascorbique (commepar exemple l'ascorbyl glucoside), le tocophérol (vitamine E) et ses esters (comme par exemple l'acétate de tocophérol), les vitamine B3 ou B10 (niacinamide et ses dérivés); les composés montrant une action éclaircissante ou dépigmentante de la peau comme par exemple le SEPIWHITE™MSH, l'arbutine, l'acide kojique, l'hydroquinone, le VEGEWHITE™, la GATULINE™, le SYNERLIGHT™, le BIOWHITE™, le PHYTOLIGHT™, le DERMALIGHT™, la CLARISKIN™, le MELASLOW™,

le DERMAWHITE™, l'ETHIOLINE, le MELAREST™, le GIGAWHITE™, l'ALBATINE™, le LUMISKIN™ ; les composés montrant une action apaisante comme le SEPICALM™ S, l'allantoïne et le bisabolol ; les agents anti-inflammatoires ; les composés montrant une action hydratante comme par exemple l'urée, les hydroxyurées, les polysaccharides, le glycérol, les polyglycérols, l'AQUAXYL™, le glycérolglucoside ; les extraits de polyphénols comme par exemple les extraits de raisin, les extraits de pin, les extraits de vin, les extraits d'olives ; les composé montrant une action amincissante ou lipolytique comme la caféine ou ses dérivés, l'ADIPOSLIM™, l'ADIPOLESS™ ; les protéines N-acylées ; les peptides N-acylés comme par exemple le MATRIXIL™ ; les acides aminés N - acylés ; les hydrolysâts partiels de protéines N-acylés ; les acides aminés ; les peptides ; les hydrolysâts totaux de protéines ; les extraits de soja, par exemple la Raffermine™ ; les extraits de blé par exemple la TENSINE™ ou la GLIADINE™ ; les extraits végétaux, tels que les extraits végétaux riches en tanins, les extraits végétaux riches en isoflavones ou les extraits végétaux riches en terpènes ; les extraits d'algues d'eau douce ou marines ; les extraits marins en général comme les coraux ; les cires essentielles ; les extraits bactériens ; les minéraux comme les dérivés de calcium, de magnésium, de cuivre, de cobalt, de zinc, de lithium, ou de manganèse ; les sels d'argent ou d'or; les céramides ; les phospholipides ; les composés montrant une action antimicrobienne ou une action purifiante, comme par exemple le LIPACIDE™ C8G, le LIPACIDE™ UG, le SEPICONTROL™ A5 ; l'OCTOPIROX™ ou le SENSIVA™ SC50 ; les composé monotrant une propriété énergisante ou stimulante comme le SEPITONIC™ M3 ou le Physiogényl™, le panthénol et ses dérivés comme le SEPICAP™ MP ; les actifs anti-age comme le SEPILIFT™ DPHP, le LIPACIDE™ PVB, le SEPIVINOL™, le SEPIVITAL™, le MANOLIVA™, le PHYTO-AGE™, le TIMECODE™ ; le SURVICODE™ ; les actifs anti-photo vieillissement ; les actifs protecteurs de l'intégrité de la jonction dermo-épidermique ; les actifs augmentant la synthèse des composants de la matrice extracellulaire comme par exemple le collagène, les élastines, les glycosaminoglycanes ; les actifs agissant favorablement sur la communication cellulaire chimique comme les cytokines ou physiques comme les intégrines ; les actifs créant une sensation de « chauffe » sur la peau comme les activateurs de la microcirculation cutanée (comme par exemple les nicotinates) ou des produits créant une sensation de « fraîcheur » sur la peau (comme par exemple le menthol et des dérivés) ; les actifs améliorant la microcirculation cutanée, par exemple les veinotoniques ; les actifs drainants ; les actifs à visée décongestionnante comme par exemple les extraits de ginko biloba, de lierre, de marron d'inde, de bambou, de ruscus, de petit houx, de *centalla asiatica,* de fucus, de romarin, de saule.

L'invention a encore pour objet un procédé de préparation d'une composition (C1) telle que définie précédemment, caractérisé en ce qu'il comprend :

une étape (a) de mélange, sous agitation, dudit tensioactif moussant, dudit agent acide (A) et de l'eau,
une étape (b) de mélange sous agitation de la combinaison préparée à l'étape (a), avec ledit polyélectrolyte anionique réticulé (P).

Les étapes a) et b) du procédé objet de la présente invention sont généralement conduites à une température comprise entre 20°C et 60 °C, plus particulièrement entre 20°C et 40 °C, sous agitation mécanique par l'intermédiaire d'un agitateur muni d'une pâle de type ancre, à une vitesse d'agitation comprise entre 50 tours/minutes et 600 tours/minutes, plus particulièrement entre 50 tours/minutes et 300 tours/minutes.

Selon un dernier aspect, l'invention a pour objet l'utilisation d'une composition (C1) telle que définie précédemment pour le nettoyage et/ou le démaquillage de la peau du visage et/ou du corps et plus particulièrement l'utilisation telle que définie précédemment pour le traitement cosmétique de l'acnée et/ou des points noirs et/ou des comédons.

Les exemples suivants illustrent l'invention sans toutefois la limiter.

**Exemple 1 :**

**1.1. Préparation d'un terpolymère de 2-méthyl 2-[(1-oxo 2-propènyl) amino] 1-propanesulfonate d'ammonium, N,N-diméthyl acrylamide et méthacrylate de lauryle tétraéthoxylé [AMPS/DMAM/MAL(4OE) 77,4/19,2/3,4 molaire], réticulé au triméthylol propanetriacrylate (TMPTA). [exemple selon l'invention]**

On charge dans un réacteur maintenu à 25°C sous agitation, 592g d'une solution aqueuse à 15% massique de 2-méthyl 2-[(1-oxo 2-propènyl) amino] 1-propanesulfonate d'ammonium dans un mélange tert-butanol/eau (97,5/2,5 en volume), 10,1g de N,N-diméthyl acrylamide, 4,2g de méthacrylate de lauryle tétraéthoxylé et 0,75g de triméthylol propanetriacrylate.

Après un temps suffisant pour atteindre une bonne homogénéisation de la solution, celle-ci est désoxygénée par barbotage d'azote chauffée à 70°C. 0,42g de peroxyde de dilauroyle sont alors ajoutés et le milieu réactionnel est ensuite maintenu pendant environ 60 minutes à 70°C puis 2 heures à 80°C.

Après refroidissement, la poudre qui s'est formée en cours de polymérisation, est filtrée et séchée pour obtenir le produit désiré, dénommé par la suite : Polyélectrolyte 1

**1.2. Préparation d'un terpolymère de 2-méthyl 2-[(1-oxo 2-propènyl) amine] 1-propanesulfonate d'ammonium, 2-hydroxyéthylacrylate et méthacrylate de lauryle tétraéthoxylé [AMPS/HEA/MAL(4OE) 77,4/19,2/3,4 molaire], réticulé au triméthylol propanetriacrylate (TMPTA) [exemple comparatif].**

En mettant en oeuvre les conditions opératoires du procédé décrit dans l'exemple 1.1 précédent, on charge dans un réacteur maintenu à 25°C sous agitation, la quantité massique nécessaire d'une solution aqueuse à 15% massique de 2-méthyl 2-[(1-oxo 2-propènyl) amino] 1-propanesulfonate d'ammonium dans un mélange tert-butanol/eau (97,5/2,5 en volume) de façon à introduire 77,4 équivalents molaires de 2-méthyl 2-[(1-oxo 2-propènyl) amino] 1-propanesulfonate d'ammonium, la quantité massique nécessaire de 2-hydroxyéthylacrylate de façon à introduire 19,2 équivalents molaires de 2-hydroxyéthylacrylate, la quantité massique nécessaire de méthacrylate de lauryle tétraéthoxylé de façon à introduire 3,4 équivalents molaires de méthacrylate de lauryle tétraéthoxylé, et la quantité massique nécessaire de triméthylol propanetriacrylate de façon à obtenir la même proportion molaire de triméthylol propanetriacrylate que dans l'exemple 1.1.

Après un temps suffisant pour atteindre une bonne homogénéisation de la solution, celle-ci est désoxygénée par barbotage d'azote chauffée à 70°C. 0,42g de peroxyde de dilauroyle sont alors ajoutés et le milieu réactionnel est ensuite maintenu pendant environ 60 minutes à 70°C puis 2 heures à 80°C.

Après refroidissement, la poudre qui s'est formée en cours de polymérisation, est filtrée et séchée pour obtenir le produit désiré, dénommé par la suite : Polyélectrolyte 2.

**1.3. Préparation d'un copolymère de 2-méthyl 2-[(1-oxo 2-propènyl) amino] 1-propanesulfonate d'ammonium et de méthacrylate de lauryle tétraéthoxylé [AMPS/MAL(4OE) 95/5 molaire], réticulé au triméthylol propanetriacrylate (TMPTA) [exemple comparatif].**

En mettant en oeuvre les conditions opératoires du procédé décrit dans l'exemple 1.1 précédent, on charge dans un réacteur maintenu à 25°C sous agitation, la quantité massique nécessaire d'une solution aqueuse à 15% massique de 2-méthyl 2-[(1-oxo 2-propènyl) amino] 1-propanesulfonate d'ammonium dans un mélange tert-butanol/eau (97,5/2,5 en volume) de façon à introduire 95 équivalents molaires de 2-méthyl 2-[(1-oxo 2-propènyl) amino] 1-propanesulfonate d'ammonium, la quantité massique nécessaire de méthacrylate de lauryle tétraéthoxylé de façon à introduire 5 équivalents molaires de méthacrylate de lauryle tétraéthoxylé, et la quantité massique nécessaire de triméthylol propanetriacrylate de façon à obtenir la même proportion molaire de triméthylol propanetriacrylate que dans l'exemple 1.1.

Après un temps suffisant pour atteindre une bonne homogénéisation de la solution, celle-ci est désoxygénée par barbotage d'azote chauffée à 70°C. 0,42g de peroxyde de dilauroyle sont alors ajoutés et le milieu réactionnel est ensuite maintenu pendant environ 60 minutes à 70°C puis 2 heures à 80°C.

Après refroidissement, la poudre qui s'est formée en cours de polymérisation, est filtrée et séchée pour obtenir le produit désiré, dénommé par la suite : Polyélectrolyte 3.

**1.4. Préparation d'un copolymère de 2-méthyl 2-[(1-oxo 2-propènyl) amino] 1-propanesulfonate d'ammonium et de 2-hydroxyéthylacrylate [AMPS/HEA 90/10 molaire], réticulé au triméthylol propanetriacrylate (TMPTA) [exemple comparatif].**

En mettant en oeuvre les conditions opératoires du procédé décrit dans l'exemple 1.1 précédent, on charge dans un réacteur maintenu à 25°C sous agitation, la quantité massique nécessaire d'une solution aqueuse à 15% massique de 2-méthyl 2-[(1-oxo 2-propènyl) amino] 1-propanesulfonate d'ammonium dans un mélange tert-butanol/eau (97,5/2,5 en volume) de façon à introduire 90 équivalents molaires de 2-méthyl 2-[(1-oxo 2-propènyl) amino] 1-propanesulfonate d'ammonium, la quantité massique nécessaire de 2-hydroxyéthylacrylate de façon à introduire 10 équivalents molaires de 2-hydroxyéthylacrylate, et la quantité massique nécessaire de triméthylol propanetriacrylate de façon à obtenir la même proportion molaire de triméthylol propanetriacrylate que dans l'exemple 1.1.

Après un temps suffisant pour atteindre une bonne homogénéisation de la solution, celle-ci est désoxygénée par barbotage d'azote chauffée à 70°C. 0,42g de peroxyde de dilauroyle sont alors ajoutés et le milieu réactionnel est ensuite maintenu pendant environ 60 minutes à 70°C puis 2 heures à 80°C.

Après refroidissement, la poudre qui s'est formée en cours de polymérisation, est filtrée et séchée pour obtenir le produit désiré, dénommé par la suite : Polyélectrolyte 4.

**Exemple 2 :**

**A- Préparation de compositions nettoyantes selon l'invention, comprenant le Polyélectrolyte 1, et de formulations comparatives.**

On verse dans un bécher, à 20°C, dans les proportions massiques indiquées dans le tableau 1, de l'eau puis progressivement et sous agitation mécanique à 50 tours/minute, le tensioactif moussant, puis progressivement l'agent épaississant, puis l'agent neutralisant (acide ou basique selon les formulations) de façon à obtenir la valeur du pH désirée. On obtient les formulations (F1), (F11), (F12) et (F13) selon l'invention et les formulations (F2) à (F10) comparatives.

| | (F1) | (F2) | (F3) | (F4) |
|---|---|---|---|---|
| Lauryl éther (2,2 OE) sulfate de sodium (27,1% M.A) | 44,3% | 44,3% | 44,3% | 44,3% |
| Amonyl™ 380 BA (1) (28,9% M.A.) | 10,4% | 10,4% | 10,4% | 10,4% |
| Amonyl™675 SB(2) (38,44% MA) | 0% | 0% | 0% | 0% |
| Eau | 44,3% | 43,6% | 42,4% | 42,5% |
| Acide lactique en solution à 45% | 0,2% | 0,2% | 0% | 0% |
| Acide citrique en solution à 10% | 0% | 0% | 0% | 0,5% |
| Triéthanolamine en solution à 50% | 0% | 0% | 0,6% | 0% |
| Soude | 0% | 0% | 0% | 0% |
| Polyélectrolyte 1 | 0,8% | 0% | 0% | 0% |
| NaCl | 0% | 1,5% | 0% | 0% |
| Capigel™98(3) | 0% | 0% | 2,3% | 2,3% |
| Gomme de xanthane | 0% | 0% | 0% | 0% |
| pH mesuré à 20°C | 5,2 | 4,7 | 7,0 | 5,5 |

**Tableau 1**

| | (F5) | (F6) | (F7) |
|---|---|---|---|
| Lauryl éther (2,2 OE) sulfate de sodium (27,1% M.A) | 44,3% | 35,0% | 35,0% |
| Amonyl™ 380 BA (1) (28,9% M.A.) | 10,4% | 0% | 0% |
| Amonyl™675 SB(2) (38,44% MA) | 0% | 5% | 5% |
| Eau | 44,0% | 59,1% | 56,6% |
| Acide lactique en solution à 45% | 0,1% | 0% | 0% |
| Acide citrique en solution à 10% | 0% | 0% | 0% |
| Triéthanolamine en solution à 50% | 0% | 0% | 0% |
| Soude | 0% | 0,1% | 0,1% |
| Polyélectrolyte 1 | 0% | 0,8% | 3,3% |
| NaCl | 0% | 0% | 0% |
| Capigel™98(3) | 0% | 0% | 0% |
| Gomme de xanthane | 1.2% | 0% | 0% |
| pH mesuré à 20°C | 5,5 | 7,2 | 7,3 |

**Tableau 1 (suite)**

| | (F8) | (F9) | (F10) |
|---|---|---|---|
| Lauryl éther (2,2 OE) sulfate de sodium (27,1% M.A) | 44,3% | 44,3% | 44,3% |
| Amonyl™ 380 BA (1) (28,9% M.A.) | 10,4% | 10,4% | 10,4% |
| Amonyl™675 SB(2) (38,44% MA) | 0% | 0% | 0% |
| Eau | 43,4% | 42,9% | 42,9% |
| Acide lactique en solution à 45% | 0,4% | 0,4% | 0,4% |
| Acide citrique en solution à 10% | 0% | 0% | 0% |
| Triéthanolamine en solution à 50% | 0% | 0% | 0% |
| Soude | 0% | 0% | 0% |
| Polyélectrolyte 2 | 1,5% | 0% | 0% |
| Polyélectrolyte 3 | 0% | 2,0% | 0% |
| Polyélectrolyte 4 | 0% | 0% | 2,0% |
| NaCl | 0% | 0% | 0% |
| Capigel™98(3) | 0% | 0% | 0% |
| Gomme de xanthane | 0% | 0% | 0% |
| pH mesuré à 20°C | 4,2 | 4,9 | 4,8 |

**Tableau 1 (suite)**

| | (F11) | (F12) | (F13) |
|---|---|---|---|
| Lauryl éther (2,2 OE) sulfate de sodium (27,1% M.A) | 0% | 0% | 0% |
| Amonyl™ 380 BA (1) (28,9% M.A.) | 10,4% | 10,4% | 0% |
| Oramix™10 (4) (54,2% MA) | 22,73% | 0% | 22,73% |
| Proteol™APL(5) (22,4% MA) | 0% | 55,0% | 0% |
| Eau | 64,57% | 32,2% | 74,87% |
| Acide lactique en solution à 45% | 0,3% | 0,4% | 0,4% |
| Acide citrique en solution à 10% | 0% | 0% | 0% |
| Triéthanolamine en solution à 50% | 0% | 0% | 0% |
| Soude | 0% | 0% | 0% |
| Polyélectrolyte 1 | 2,0% | 2,0% | 2,0% |
| NaCl | 0% | 0% | 0% |
| Capigel™98(3) | 0% | 0% | 0% |
| Gomme de xanthane | 0% | 0% | 0% |
| pH mesuré à 20°C | 5,1 | 6,3 | 5,1 |

**Tableau 1 (suite)**

% M.A. : % massique en matière active.

(1) : AMONYL™ 380 BA est une Cocamidopropyl betaïne, commercialisée par la société SEPPIC.
(2) : AMONYL™ 675SB est une Cocoamidopropyl hydroxy sultaïne, commercialisée par la société SEPPIC.
(3) : CAPIGEL™ 98 est un copolymère d'acrylates de type HASE commercialisé par la société SEPPIC.
(4): ORAMIX™NS 10 est une composition moussante comprenant de l'octanoylpolyglucoside et du décylpolyglucoside commercialisée par la société SEPPIC
(5) : PROTEOL™APL est une composition moussante comprenant un sel de sodium de cocoyl amino-acides provenant du jus de pomme, commercialisée par la société SEPPIC.

**B- Mise en évidence des propriétés et des caractéristiques des formulations nettoyantes selon l'invention comparativement à celles de l'état de la technique.**

Les formulations (F1) à (F13) précédement préparées sont ensuite évaluées comme suit :

- Mesure de leur pH à 20°C ;
- Mesure de leur viscosité à 20°C au moyen d'un viscosimètre de type Brookfield LVT à une vitesse de 6 tours/minutes, muni du mobile adapté ;
- Evaluation visuelle de leur aspect ;
- Evaluation de la taille et de la stabilité des bulles de la mousse générées par chacune d'entre elles selon le portocole expérimental exposé ci-dessous.

Protocole expérimental pour l'évaluation de la taille et de la stabilité des bulles de la mousse générées dans les formulations (F1) à (F13)

**i)** Préparation d'une solution moussante (**S**)

Pour chacune des formulations (F1) à (F13) à tester, on prépare une solution aqueuse (S) de 500 $cm^3$ à 1% massique en matière active de la formulation à tester, dans de l'eau déminéralisée enrichie de 3 millimoles d'ions calcium, (correspondant à la préparation d'une eau à 30° de dureté calcique selon la norme NFT 73-047).

**ii)** Préparation de la solution d'essai

Pour chacune des formulations (F1) à (F13) à tester, on prélève 25 $cm^3$ de la solution (S) correspondante, dans un bêcher de 100 $cm^3$ de forme haute, qui est ensuite placé dans un bain-Marie thermostaté pendant 30 minutes, pour atteindre une température de 40°C +/-1°C.

**iii)** Foisonnement de la solution d'essai

Lorsque la solution d'essai (S) est stabilisée à une température de 40°C, on l'agite dans le bêcher au moyen d'un agitateur de type Rayneri muni d'une turbine centripète de taille adaptée à une vitesse de 3000 tours/minute, pendant une durée de deux minutes de façon à générer une mousse.

**iv)** Observation de la mousse générée par la solution d'essai

La mousse formée est transvasée dans une boite de pétri (5,3 cm de diamètre x 1,2 cm de hauteur), jusqu'à débordement puis étalée sur toute la surface de la boite. Cette boite à pétri est placée sous l'optique d'un microscope électronique de marque NI-KON OPTIPHOP-2, muni d'une caméra numérique NIKON DXM 1200 et associé à un ordinateur équipé du logiciel NIKON ACT-1 ; le grossissement étant de x 40. La mise est point est effectuée, et une première photographie est prise coïncidant au déclenchement d'un chronomètre. De nouvelles photographies sont ensuite prises après une durée de 10 minutes et de 20 minutes suivant le déclenchement du chronomètre.

**v)** Expression des résultats

Les opérations décrites aux paragraphes ii) à iv) précédents sont reproduits pour chacune des formulations (F1) à (F13) de façon à disposer d'une population statistique significative. Pour chaque photographie prise pour une formulation donnée et à un temps donné, l'expérimentateur identifie le nombre total de bulles présentes sur la photographie ($N_T$), et mesure pour chacune des bulles le diamètre correspondant à l'aide de l'échelle micrométrique présente et mise à disposition par le logiciel NIKON ACT-1.
L'expérimentateur relève ensuite :

- Le nombre de bulles dont le diamètre est inférieur ou égale à 150 micromètres (N1) pour chacune des photographies relatives à une formulation donnée ;
- Le nombre de bulles dont le diamètre est supérieur à 150 micromètres et inférieur ou égal à 450 micromètres (N2) ;
- Le nombre de bulles dont le diamètre est supérieur à 450 micromètres (N3).

L'expérimentateur calcule ensuite pour chaque photographie les rapports suivants :

- R1 = (N1/NT) x 100
- R2 = (N2/NT) x 100
- R3 = (N3/NT) x 100

L'expérimentateur calcule la moyenne des rapports R1, R2 et R3 à t = 0, sont nommés resepctivement $R1m_0$ $R2m_0$ et $R3m_0$, puis la moyenne des rapports R1, R2 et R3 à t = 10 minutes, sont nommés resepctivement $R1m_{10}$, $R2m_{10}$ et $R3m_{10}$, et la moyenne des rapports R1, R2 et R3 à t = 20 minutes, sont nommés respectivement $R1_{m20}$, $R2m_{20}$ et $R3m_{20}$.

Les résultats de ces évaluations sont consignés dans le tableau 2 suivant.

| | (F1) | (F2) | (F3) | (F4) |
|---|---|---|---|---|
| pH | 5,2 | 4,7 | 7,0 | 5,5 |
| Viscosité (en mPas) | 4529 | 9800 | 6200 | 430 |
| Aspect (visuel) | Liquide Homogène | Liquide homogène | Liquide homogène | Liquide Homogène |
| Répartition des diamètres des bulles de mousse à T = 0 | | | | |
| $R1m_0$ | 91,0 % | 73,5% | 83,9% | 87,8% |
| $R2m_0$ | 9% | 26,5% | 16,1% | 12,2% |
| $R3m_0$ | 0% | 0% | 0% | 0% |
| Répartition des diamètres des bulles de mousse à T = 10 minutes | | | | |
| $R1m_{10}$ | 78% | 50,0% | 50,0% | 53,3% |
| $R2m_{10}$ | 22% | 50,0% | 47,1% | 44,4% |
| $R3m_{10}$ | 0% | 0% | 2,9% | 2,3% |
| Répartition des diamètres des bulles de mousse à T = 20 minutes | | | | |
| $R1m_{20}$ | 76,5% | 35,3% | 52,9% | 50% |
| $R2m_{20}$ | 23,5% | 41,7% | 29,4% | 40,9% |
| $R3m_{20}$ | 0% | 17,6% | 17,7% | 9,1% |

**Tableau 2**

| | (F5) | (F6) | (F7) |
|---|---|---|---|
| pH | 5,5 | 7,2 | 7,3 |
| Viscosité (en mPas) | 6400 | 70 | 270 000 |
| Aspect (visuel) | Liquide Hétérogène légèrement filant | Liquide homogène | Compact non liquide |
| Répartition des diamètres des bulles de mousse à T = 0 | | | |
| $R1m_0$ | 85,5% | 75,4% | 95,4% |
| $R2m_0$ | 14,5% | 24,6% | 4,5% |
| $R3m_0$ | 0% | 0% | 0% |
| Répartition des diamètres des bulles de mousse à T = 10 minutes | | | |
| $R1m_{10}$ | 61,7% | 51,3% | 62,0% |
| $R2m_{10}$ | 34,0% | 46,3% | 62,0% |
| $R3m_{10}$ | 4,3% | 2,4% | 0,0% |
| Répartition des diamètres des bulles de mousse à T = 20 minutes | | | |
| $R1m_{20}$ | 31,8% | 34,8% | 48,5% |
| $R2m_{20}$ | 50,0% | 43,5% | 33,3% |
| $R3m_{20}$ | 18,2% | 21,7% | 18,2% |

**Tableau 2 (suite)**

| | (F8) | (F9) | (F10) |
|---|---|---|---|
| pH | 4,2 | 4,9 | 4,8 |
| Viscosité (en mPas) | 3300 | 80 | 100 |
| Aspect (visuel) | Liquide homogène | Liquide homogène | Liquide Homogène voilé |
| Répartition des diamètres des bulles de mousse à T = 0 | | | |
| $R1m_0$ | 97,3% | n.m | n.m |
| $R2m_0$ | 2,7% | n.m | n.m |
| $R3m_0$ | 0% | n.m | n.m |
| Répartition des diamètres des bulles de mousse à T = 10 minutes | | | |
| $R1m_{10}$ | 58,7% | n.m | n.m |
| $R2m_{10}$ | 39,1% | n.m | n.m |
| $R3m_{10}$ | 2,2% | n.m | n.m |

**Tableau 2 (suite)**

| | (F11) | (F12) | (F13) |
|---|---|---|---|
| pH | 5,1 | 6,3 | 5,1 |
| Viscosité (en mPas) | 3885 | 2010 | 7460 |
| Aspect (visuel) | Liquide homogène | Liquide homogène | Liquide Homogène |
| Répartition des diamètres des bulles de mousse à T = 0 | | | |
| $R1m_0$ | 94,9% | 95,5% | 98,8% |
| $R2m_0$ | 5,1% | 4,5% | 5,1% |
| $R3m_0$ | 0% | 0% | 0% |
| Répartition des diamètres des bulles de mousse à T = 10 minutes | | | |
| $R1m_{10}$ | 72,9% | 93,6% | 96,1% |
| $R2m_{10}$ | 27,1% | 6,4% | 3,9% |
| $R3m_{10}$ | 0% | 0% | 0% |

**Tableau 2 (suite)**

n.m. : non mesuré.

**3) Analyse des résultats**

Les résultats sont jugés satisfaisants lorsque, l'aspect de la formulation est liquide, la viscosité est comprise entre 2 000 mPa.s et 30 000 mPa.s, lorsque dans la mousse générée par la formulation, la proportion du nombre de bulles dont le diamètre moyen est inférieur ou égal à 150 micromètres après 10 minutes suivant le foisonnement (mesurée par $R1m_{10}$) est supérieure à 70%.

La formulation (F1) selon l'invention se caractérise par un aspect liquide, une viscosité mesurée à 4529 mPa.s et la mousse formée par (F1) montre une proportion en nombre de bulles dont le diamètre moyen inférieur ou égal à 150 micromètres après 10 minutes suivant le foisonnement égale à 78%. On observe également que cette mousse est très stable, puisque après 20 minutes suivant le foisonnement, la proportion en nombre de bulles dont le diamètre moyen est inférieur ou égal à 150 micromètres est égale à 76,5%.

Pour les mousses obtenues à partir des formulations (F6) et (F7), qui sont divulguées et enseignées par la publication internationale WO 2011/030044, et qui se caractérisent par une valeur de pH supérieure à 7,0, on observe une proportion en nombre de bulles dont le diamètre moyen est inférieur ou égal à 150 micromètres après 10 minutes suivant le foisonnement respectivement égale à 51,3 % pour (F6) et à 62% pour (F7). Dans la mousse générée par la formulation (F6), il faut également noter une proportion en nombre de bulles dont le diamètre moyen est supérieur à 450 micromètres après 10 minutes suivant le foisonnement égale à 2,4%.

Pour la mousse obtenue à partir de la formulation (F2), épaissie par l'utilisation de 1,5% de chlorure de sodium, on observe une proportion en nombre de bulles dont le diamètre moyen est inférieur ou égal à 150 micromètres après 10 minutes suivant le foisonnement égale à 50%.

Pour les mousses formées par les formulations (F3) et (F4), épaissies par l'utilisation de Capigel™ 98, polymère de type HASE, on observe une proportion en nombre de bulles dont le diamètre moyen est inférieur ou égal à 150 micromètres après 10 minutes suivant le foisonnement égale respectivement à 50% et à 53,3%, ainsi qu'une proportion non nulle de bulles dont les diamètres moyen est supérieur à 450 micromètres après 10 minutes suivant le foisonnement.

Pour la mousse obtenue à partir de la formulation (F5), épaissie par l'utilisation de 2,25% de gomme de xanthane, on observe une proportion en nombre une proportion en nombre de bulles dont le diamètre moyen est inférieur ou égal à 150 micromètres après 10 minutes suivant le foisonnement égale à 61,7% et une proportion égale à 4,3% de bulles dont les diamètres moyen est supérieur à 450 micromètres après 10 minutes suivant le foisonnement.

La formulation (F8) comprenant le Polyélectrolyte 2 se caractérise par un aspect liquide homogène, une viscosité mesurée à 3300 mPa.s, et la mousse formée par (F8) montre une proportion du nombre de bulles dont le diamètre

moyen inférieur ou égal à 150 micromètres après 10 minutes suivant le foisonnement égale à 58,7%.

La formulation (F9) comprenant le Polyélectrolyte 3 se caractérise par un aspect liquide homogène et une viscosité mesurée à 80 mPa.s. La formulation (F9) ne permet pas d'obtenir le niveau de viscosité requis, à savoir une valeur minimale de la viscosité de 2 000 mPa.s.

La formulation (F10) comprenant le Polyélectrolyte 4 se caractérise par un aspect liquide homogène et une viscosité mesurée à 100 mPa.s. La formulation (F10) ne permet pas d'obtenir le niveau de viscosité requis, à savoir une valeur minimale de la viscosité de 2 000 mPa.s.

Les formulations (F11), (F12) et (F13) selon l'invention se caractérisent par un aspect liquide et homogène, une viscosité mesurée supérieure à 2000 mPa.s et les mousses formées par les formulations (F11), (F12) et (F13) montrent une proportion en nombre de bulles dont le diamètre moyen inférieur ou égal à 150 micromètres après 10 minutes suivant le foisonnement supérieures à 70%.

La mise en perspective de ces mesures indique clairement que l'amélioration propriétés moussantes d'une formulation nettoyante à usage topique, comprenant au moins un agent tensioactif moussant et présentant une valeur de pH supérieure ou égale à 4,0 et inférieure ou égale à 6,5, est apportée par la mise en oeuvre du procédé selon l'invention qui consiste à incorporer dans ladite formulation nettoyante une quantité efficace d'un polyélectrolyte anionique réticulé (**P**) tel que décrit précédemment.

## Revendications

1. Procédé pour améliorer les propriétés moussantes d'une formulation nettoyante à usage topique, de pH supérieur ou égal à 4,0 et inférieur ou égal à 6,5 et comprenant au moins un agent tensioactif moussant, ledit procédé étant **caractérisé en ce que** l'on incorpore dans ladite formulation nettoyante à usage topique, une quantité efficace d'un polyélectrolyte anionique réticulé (P) issu de la polymérisation, en présence d'au moins un agent de réticulation, d'au moins un monomère possédant une fonction acide fort, ledit monomère étant l'acide 2-méthyl 2-[(1-oxo 2-propènyl) amino] 1-propanesulfonique partiellement ou totalement salifié, avec au moins un monomère neutre choisi parmi les N,N-dialkyl acrylamides, dans lesquels chacun des groupes alkyle comportent entre un et quatre atomes de carbones, et au moins un monomère de formule (I) :

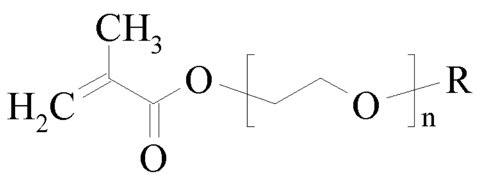

(I)

dans laquelle R représente un radical alkyle linéaire ou ramifié comportant de huit à vingt atomes de carbone et n représente un nombre supérieur ou égal à un et inférieur ou égal à vingt.

2. Procédé tel que défini à la revendication 1, **caractérisé en ce que** ledit polyélectrolyte anionique réticulé (P) comporte pour 100% molaire :

   - De 20% molaire à 80% molaire d'unités monomériques issues du monomère comportant une fonction acide fort partiellement ou totalement salifiée ;
   - De 15% molaire à 75% molaire d'unités monomériques issues d'un monomère neutre choisi parmi les N,N-dialkyl acrylamides, dans lesquels chacun des groupes alkyle comportent entre un et quatre atomes de carbones;
   - De 0,5% à 5% molaire d'unités monomériques issues d'un monomère de formule (I) telle que définie précédemment.

3. Procédé tel que défini à l'une quelconque des revendications 1 ou 2, **caractérisé en ce que** dans ledit polyélectrolyte anionique réticulé (P), ledit monomère neutre est le N,N-diméthyl acrylamide.

4. Procédé tel que défini à l'une quelconque des revendications 1 à 3, **caractérisé en ce que** dans ledit polyélectrolyte anionique réticulé (P), ledit monomère de formule (I) est le méthacrylate de lauryle tétraéthoxylé.

5. Procédé tel que défini à l'une quelconque des revendications 1 à 4, **caractérisé en ce que** ledit polyélectrolyte anionique réticulé (P) est un terpolymère de l'acide 2-méthyl 2-[(1-oxo 2-propènyl) amino] 1-propanesulfonique

partiellement salifié sous forme de sel d'ammonium, du N,N-diméthyl acrylamide et du méthacrylate de lauryle tétraéthoxylé, réticulé au triméthylol propanetriacrylate.

**6.** Procédé tel que défini à l'une quelconque des revendications 1 à 5, **caractérisé en ce que** ledit polyélectrolyte anionique réticulé (P) comporte pour 100% molaire :

- de 60% molaire à 80% molaire d'unités monomériques issues de l'acide 2-méthyl 2-[(1-oxo 2-propènyl) amino] 1-propanesulfonique partiellement salifié sous forme d'ammonium,
- de 15% molaire à 39,5% molaire d'unités monomériques issues du N,N-diméthyl acrylamide, et
- de 0,5% molaire à 5% molaire d'unités monomériques issues du méthacrylate de lauryle tétraéthoxylé

**7.** Procédé tel que défini à l'une ou quelconque des revendications 1 à 6, pour lequel la formulation nettoyante à usage topique comprend au moins un agent tensioactif moussant anionique de formule (II) :

$$R_2\text{-}O\text{-}(CH_2\text{-}CH_2\text{-}O)_pSO_3\text{-}X \qquad (II)$$

dans laquelle $R_2$ représente un radical hydrocarboné aliphatique, saturé ou insaturé, linéaire ou ramifié, comportant de 6 à 22 atomes de carbone, p représente un nombre décimal compris entre 1 et 10, de préférence entre 2 et 4, X représente le cation d'un métal alcalin ou d'un métal alcalino-terreux, l'ion ammonium, l'ion hydroxyéthyl ammonium, l'ion tris(hydroxyéthyl) ammonium, ou un mélange de composés de formule (II).

**8.** Procédé tel que défini à l'une ou quelconque des revendications 1 à 6, pour lequel la formulation nettoyante à usage topique comprend au moins un agent tensioactif moussant amphotère de formule (III) :

$$R_3\text{-}C(O)\text{-}NH(CH_2)_q\text{-}N^+(R_4)(R_5)\text{-}(CH_2)_s\text{-}CO_2^- \qquad (III)$$

dans laquelle $R_3$ représente un radical hydrocarboné aliphatique, saturé ou insaturé, linéaire ou ramifié, comportant de 7 à 21 atomes de carbone, $R_4$ et $R_5$ représentent indépendamment l'un de l'autre un radical aliphatique, saturé ou insaturé, linéaire ou ramifié, éventuellement substitué avec un groupe hydroxyle, comportant de 1 à 4 atomes de carbone, q représente un nombre entier compris entre 2 et 6, et s représente un nombre entier égal à 1 ou à 2 ou un mélange de composés de formule (III).

**9.** Procédé tel que défini à l'une ou quelconque des revendications 1 à 8, pour lequel la formulation nettoyante à usage topique comprend un mélange d'au moins un composé de formule (II) telle que définie précédemment avec au moins un composé de formule (III) telle que définie précédemment.

**10.** Procédé tel que défini à l'une ou quelconque des revendications 1 à 6, pour lequel la formulation nettoyante à usage topique comprend au moins un composé de formule (IV) :

$$R6\text{-}O\text{-}(S)_y\text{-}H \qquad (IV)$$

dans laquelle :

y est un nombre décimal compris entre 1,05 et 2,
S représente le reste d'un sucre réducteur choisi parmi le glucose, le xylose ou l'arabinose.
$R_6$ représente un radical choisi parmi les radicaux n-octyle, n-décyle, n-dodécyle, n-tétradécyle, n-hexadécyle, ou un mélange de composés de formule (IV).

**11.** Procédé tel que défini à l'une ou quelconque des revendications 1 à 10, **caractérisé en ce que** le rapport massique agent tensioactif moussant sur polyélectrolyte anionique réticulé (P) est compris entre 1/10 et 40/1.

**12.** Composition (C1) **caractérisée en ce qu'**elle comprend pour 100% de sa masse :

- De 0,05% à 2% massique d'au moins un polyélectrolyte anionique réticulé (P) issu de la polymérisation d'au moins un monomère possédant une fonction acide fort, ledit monomère étant l'acide 2-méthyl 2-[(1-oxo 2-propènyl) amino] 1-propanesulfonique, partiellement ou totalement salifiée, avec au moins un monomère neutre choisi parmi les N,N-dialkyl acrylamides, dans lesquels chacun des groupes alkyle comportent entre un et quatre atomes de carbones, et au moins un monomère de formule (I) :

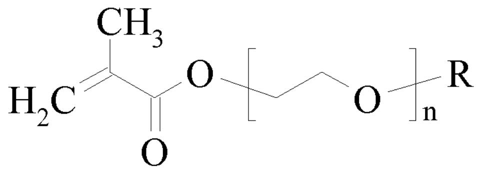

(I)

dans laquelle R représente un radical alkyle linéaire ou ramifié comportant de huit à vingt atomes de carbone et n représente un nombre supérieur ou égal à un et inférieur ou égal à vingt, en présence d'au moins un agent de réticulation ;
- De 10% à 50% massique d'au moins un tensioactif moussant sélectionné parmi les agents tensioactifs anioniques, les agents tensioactifs non ioniques et les agents tensioactifs amphotères ;
- De 0,01% à 10% massique d'au moins un agent acide (A) sélectionné parmi les $\alpha$-hydroxy acides et les $\beta$-hydroxy acides libres, partiellement ou totalement salifiés ;
- De 89,94% à 38% massique d'eau,

et **en ce que** son pH est supérieur ou égal à 4,0 et inférieur ou égal à 6,5.

**13.** Composition (C1) telle que définie à la revendication 12, **caractérisée en ce que** le rapport massique agent tensioactif moussant sur polyélectrolyte anionique réticulé (P) est compris entre 1/10 et 40/1.

**14.** Composition (C1) telle que définie à l'une ou quelconques des revendications 12 ou 13, **caractérisée en ce que** l'agent acide (A) est sélectionné parmi les éléments du groupe constitué par l'acide lactique, l'acide citrique, l'acide glycolique, l'acide gluconique, l'acide tartrique, l'acide malique, l'acide salicylique.

**15.** Utilisation d'une composition (C1) telle que définie à l'une ou quelconque des revendications 12 à 14 pour le nettoyage et/ou le démaquillage de la peau du visage et/ou du corps.

**Patentansprüche**

**1.** Verfahren zum Verbessern des Schaumvermögens einer Reinigungsformulierung zur topischen Anwendung mit einem pH-Wert größer oder gleich 4,0 und kleiner oder gleich 6,5 und zumindest ein schaumerzeugendes Tensid umfassend, wobei das besagte Verfahren **dadurch gekennzeichnet ist, dass** man in die besagte Reinigungsformulierung zur topischen Anwendung eine wirksame Menge eines vernetzten anionischen Polyelektrolyts (P) einbringt, das aus der Polymerisation hervorgeht, in Gegenwart zumindest eines Vernetzungsmittels, zumindest eines stark säurefunktionalen Monomers, wobei das besagte Monomer die ganz oder teilweise in Salz überführte 2-Methyl-2-[(1-oxo-2-propenyl)amino]-1-Propansulfonsäure ist, mit zumindest einem neutralen Monomer, das aus den N,N-Dialkylacrylamiden ausgewählt wird, wobei jede der Alkyl-Gruppen ein bis vier Kohlenstoffatome umfasst, und zumindest ein Monomer der Formel (I):

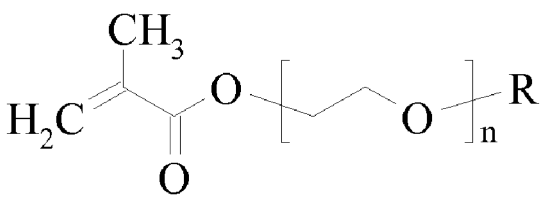

(I)

wobei R einen linearen oder verzweigten Alkylrest darstellt, der acht bis zwanzig Kohlenstoffatome umfasst, und n eine Zahl größer oder gleich eins und kleiner oder gleich zwanzig darstellt.

**2.** Verfahren nach Anspruch 1, **dadurch gekennzeichnet, dass** der besagte vernetzte anionische Polyelektrolyt (P) für 100 Mol-% folgendes umfasst:

- 20 Mol-% bis 80 Mol-% an Monomereinheiten, die aus dem Monomer hervorgegangen sind, eine ganz oder teilweise in Salz überführte starke funktionelle Säuregruppe umfassend;

- 15 Mol-% bis 75 Mol-% an Monomereinheiten, die aus einem neutralen Monomer hervorgegangen sind, das aus den N,N-Dialkylacrylamiden ausgewählt wird, wobei jede der Alkyl-Gruppen ein bis vier Kohlenstoffatome umfasst;
- 0,5 Mol-% bis 5 Mol-% an Monomereinheiten, die aus einem Monomer der Formel (I) hervorgegangen sind, wie sie zuvor definiert wurde.

**3.** Verfahren nach einem der Ansprüche 1 oder 2, **dadurch gekennzeichnet, dass** das besagte neutrale Monomer im vernetzten anionischen Polyelektrolyt (P) das N,N-Dimethylacrylamid ist.

**4.** Verfahren nach irgendeinem der Ansprüche 1 bis 3, **dadurch gekennzeichnet, dass** das besagte Monomer der Formel (I) im vernetzten anionischen Polyelektrolyt (P) das tetraethoxylierte Laurylmethacrylat ist.

**5.** Verfahren nach irgendeinem der Ansprüche 1 bis 4, **dadurch gekennzeichnet, dass** der besagte vernetzte anionische Polyelektrolyt (P) ein Terpolymer der teilweise in Salz überführten 2-Methyl-2-[(1-oxo-2-propenyl)amino]-1-Propansulfonsäure in Form von Ammoniumsalz, des N,N-Dimethylacrylamids und des tetraethoxylierten Laurylmethacrylats ist, das mit dem Trimethylolpropantriacrylat vernetzt ist.

**6.** Verfahren nach irgendeinem der Ansprüche 1 bis 5, **dadurch gekennzeichnet, dass** der besagte vernetzte anionische Polyelektrolyt (P) für 100 Mol-% folgendes umfasst:

- 60 Mol-% bis 80 Mol-% an Monomereinheiten, die aus der teilweise in Salz überführten 2-Methyl-2-[(1-oxo-2-propenyl)amino]-1-Propansulfonsäure in Form von Ammoniumsalz hervorgegangen sind,
- 15 Mol-% bis 39,5 Mol-% an Monomereinheiten, die aus dem N,N-Dimethylacrylamid hervorgegangen sind, und
- 0,5 Mol-% bis 5 Mol-% an Monomereinheiten, die aus dem tetraethoxylierten Laurylmethacrylat hervorgegangen sind.

**7.** Verfahren nach irgendeinem der Ansprüche 1 bis 6, wobei die Reinigungsformulierung zur topischen Anwendung zumindest ein anionisches schaumerzeugendes Tensid der Formel (II) umfasst:

$$\mathbf{R_2\text{-}O\text{-}(CH_2\text{-}CH_2\text{-}O)_pSO_3\text{-}X} \qquad \mathbf{(II)}$$

wobei $R_2$ einen linearen oder verzweigten, gesättigten oder nicht gesättigten aliphatischen Kohlenwasserstoffrest darstellt, der 6 bis 22 Kohlenstoffatome umfasst, p eine Dezimalzahl zwischen 1 und 10, und vorzugsweise zwischen 2 und 4 darstellt, X das Kation eines Alkalimetalls oder eines Erdalkalimetalls darstellt, das Ammoniumion, das Hydroxymethylammonium-Ion, das Tris(Hydroxymethyl)-Ammonium-Ion, oder eine Mischung von Verbindungen der Formel (II) ist.

**8.** Verfahren nach irgendeinem der Ansprüche 1 bis 6, wobei die Reinigungsformulierung zur topischen Anwendung zumindest ein amphoteres schaumerzeugendes Tensid der Formel (III) umfasst:

$$\mathbf{R_3\text{-}C(O)\text{-}NH(CH_2)_q\text{-}N^+(R_4)(R_5)\text{-}(CH_2)_s\text{-}CO_2^-} \qquad \mathbf{(III)}$$

wobei $R_3$ ein einen linearen oder verzweigten, gesättigten oder nicht gesättigten aliphatischen Kohlenwasserstoffrest darstellt, der 7 bis 21 Kohlenstoffatome umfasst, $R_4$ und $R_5$ unabhängig voneinander einen linearen oder verzweigten, gesättigten oder nicht gesättigten aliphatischen Rest darstellt, der eventuell mit einer Hydroxygruppe substituiert wird, 1 bis 4 Kohlenstoffatome umfassend, q eine ganze Zahl zwischen 2 und 6 darstellt, und s eine ganze Zahl gleich 1 oder 2 oder eine Mischung von Verbindungen der Formel (III) darstellt.

**9.** Verfahren nach einem oder irgendeinem der Ansprüche 1 bis 8, wobei die Reinigungsformulierung zur topischen Anwendung eine Mischung von zumindest einer Verbindung der zuvor definierten Formel (II) mit zumindest einer Verbindung der zuvor definierten Formel (III) umfasst.

**10.** Verfahren nach einem oder irgendeinem der Ansprüche 1 bis 6, wobei die Reinigungsformulierung zur topischen Anwendung zumindest ein amphoteres schaumerzeugendes Tensid der Formel (IV) umfasst:

$$\mathbf{R_6\text{-}O\text{-}(S)_y\text{-}H} \qquad \mathbf{(IV)}$$

wobei:

y eine Dezimalzahl zwischen 1,05 und 2 ist,
S den Rest eines reduzierenden Zuckers ist, der aus Glucose, Xylose oder Arabinose ausgewählt wird.
$R_6$ stellt einen Rest dar, der aus den Resten n-Octyl, n-Decyl, n-Dodecyl, n-Tetradecyl, n-Hexadecyl, oder einer Mischung von Verbindungen der Formel (IV) ausgewählt wird.

**11.** Verfahren nach einem oder irgendeinem der Ansprüche 1 bis 10, **dadurch gekennzeichnet, dass** das Gewichtsverhältnis schaumerzeugendes Tensid auf dem vernetzten anionischen Polyelektrolyt (P) zwischen 1/10 und 40/1 liegt.

**12.** Zusammensetzung (C1), **dadurch gekennzeichnet, dass** sie für 100% ihres Gewichts folgendes umfasst:

- 0,5 bis 2 Gew.-% zumindest eines vernetzten anionischen Polyelektrolyts (P), das aus der Polymerisierung zumindest eines Monomers hervorgeht, das eine starke Säurefunktion aufweist, wobei das besagte Monomer die ganz oder teilweise in Salz überführte 2-Methyl-2-[(1-oxo-2-propenyl)amino]-1-Propansulfonsäure ist, mit zumindest einem neutralen Monomer, das aus den N,N-Dialkylacrylamiden ausgewählt wird, wobei jede der Alkyl-Gruppen ein bis vier Kohlenstoffatome umfasst, und zumindest ein Monomer der Formel (I) :

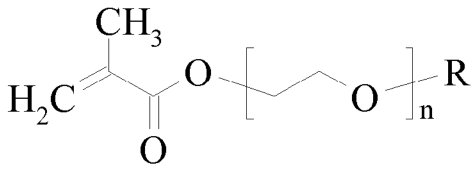

(I)

wobei R einen linearen oder verzweigten Alkylrest darstellt, der acht bis zwanzig Kohlenstoffatome umfasst, und n eine Zahl größer oder gleich eins und kleiner oder gleich zwanzig darstellt, in Gegenwart zumindest eines Vernetzungsmittels;
- 10 bis 50 Gew.-% zumindest eines schaumerzeugenden Tensids, das aus anionischen schaumerzeugenden Tensiden, nicht ionischen Tensiden und amphoteren Tensiden ausgewählt wird;
- 0,01 bis 10 Gew.-% zumindest eines sauren Mittels (A), das aus den teilweise oder ganz in Salz überführten $\alpha$-Hydroxysäuren und den freien $\beta$-Hydroxysäuren ausgewählt wird;
- 89,94 bis 38 Gew.-% Wasser,

und dadurch, dass ihr pH-Wert größer oder gleich 4,0 und kleiner oder gleich 6,5 ist.

**13.** Zusammensetzung (C1) nach Anspruch 12, **dadurch gekennzeichnet, dass** das Gewichtsverhältnis schaumerzeugendes Tensid auf dem vernetzten anionischen Polyelektrolyt (P) zwischen 1/10 und 40/1 liegt.

**14.** Zusammensetzung (C1) nach einem oder irgendeinem der Ansprüche 12 oder 13, **dadurch gekennzeichnet, dass** das saure Mittel (A) aus den Elementen der Gruppe ausgewählt wird, die durch Milchsäure, Zitronensäure, Glykolsäure, Glukonsäure, Weinsäure, Äpfelsäure, Salicylsäure gebildet wird.

**15.** Verwendung einer Zusammensetzung (C1) nach einem oder irgendeinem der Ansprüche 12 bis 14 zum Reinigen und/ oder Abschminken der Haut des Gesichts und/ oder des Körpers.

## Claims

**1.** Process for improving the foaming properties of a cleaning formulation for topical use, with pH greater than or equal to 4.0 and less than or equal to 6.5 and comprising at least one foaming surfactant, said process being **characterised in that** an effective quantity of a cross-linked anionic polyelectrolyte (P) derived from polymerisation is added to said cleaning formulation for topical use, in the presence of at least one cross-linking agent, at least one monomer with a strong acid function, said monomer being partially or completely salified 2-methyl 2-[(1-oxy 2-propenyl) amino] 1-propanesulfonic acid, with at least one neutral monomer chosen from among N, N-dialkyl acrylamides, in which each of the alkyl groups comprises between one and four carbon atoms, and at least one monomer with formula (I):

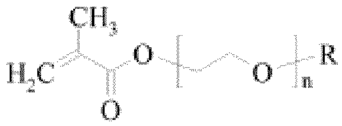

(I)

where R represents a linear or ramified alkyl radical comprising from eight to twenty carbon atoms and n represents a number greater than or equal to one and less than or equal to twenty.

2. Process as defined in claim 1, **characterised in that** said cross-linked anionic polyelectrolyte (P) comprises the following for 100% molar:

- from 20% molar to 80% molar of monomeric units derived from the monomer comprising a partially or completely salified strong acid function;
- from 15% molar to 75% molar of monomeric units derived from a neutral monomer chosen from among N, N-dialkyl acrylamides, in which each of the alkyl groups comprises between one and four carbon atoms;
- from 0.5% molar to 5% molar of monomeric units derived from a monomer with formula (I) as defined above.

3. Process as defined in either claim 1 or 2, **characterised in that** said neutral monomer in said cross-linked anionic polyelectrolyte (P) is N, N-dimethyl acrylamide.

4. Process as defined in any one of claims 1 to 3, **characterised in that** said monomer with formula (I) in said cross-linked anionic polyelectrolyte (P) is tetraethoxylated lauryl methacrylate.

5. Process as defined in any one of claims 1 to 4, **characterised in that** said cross-linked anionic polyelectrolyte (P) is a terpolymer of 2-methyl 2-[(1-oxo 2-propenyl) amino] 1-propanesulfonic acid partially salified in the form of an ammonium salt, N,N-dimethyl acrylamide and tetraethoxylated lauryl methacrylate, cross-linked with trimethylol propanetriacrylate.

6. Process as defined in any one of claims 1 to 5, **characterised in that** said cross-linked anionic polyelectrolyte (P) comprises the following for 100% molar:

- from 60% molar to 80% molar of monomeric units derived from partially salified 2-methyl 2-[(1-oxo 2-propenyl) amino] 1-propanesulfonic acid, in ammonium form;
- from 15% molar to 39.5% molar of monomeric units derived from N, N-dimethyl acrylamide; and
- from 0.5% molar to 5% molar of monomeric units derived from tetraethoxylated lauryl methacrylate.

7. Process as defined in any one of claims 1 to 6, for which the cleaning formulation for topical use comprises at least one anionic foaming surfactant with formula (II):

$$\mathbf{R_2\text{-}O\text{-}(CH_2\text{-}CH_2)O)_pSO_3\text{-}X} \qquad \textbf{(II)}$$

where $R_2$ represents a saturated or unsaturated, linear or ramified aliphatic hydrocarbon radical, comprising 6 to 22 carbon atoms, p represents a decimal number between 1 and 10, preferably between 2 and 4, X represents the cation of an alkaline metal or an alkaline-earth metal, the ammonium ion, the hydroxyethyl ammonium ion, the tris(hydroxyethyl) ammonium ion, or a mix of compounds with formula (II).

8. Process as defined in any one of claims 1 to 6, for which the cleaning formulation for topical use comprises at least one amphoteric foaming surfactant with formula (III):

$$\mathbf{R_3\text{-}C(O)\text{-}NH(CH_2)_q\text{-}N^+(R_4)(R_5)\text{-}(CH_2)_s\text{-}CO_2^-} \qquad \textbf{(III)}$$

where $R_3$ represent a saturated or unsaturated, linear or ramified aliphatic hydrocarbon radical, comprising 7 to 21 carbon atoms, $R_4$ and $R_5$ represent a saturated or unsaturated, linear or ramified aliphatic radical independently of each other, possibly substituted with a hydroxyl group, comprising 1 to 4 carbon atoms, q represents an integer number between 2 and 6, and s represents an integer number equal to 1 or 2 or a mix of compounds with formula (III).

9. Process as defined in any one of claims 1 to 8, for which the cleaning formulation for topical use comprises a mix

of at least one compound with formula (II) as defined above with at least one compound with formula (III) as defined above.

**10.** Process as defined in any one of claims 1 to 6, for which the cleaning formulation for topical use comprises at least one compound with formula (IV).

$$R_6\text{-O-(S)}_y\text{-H} \qquad (IV)$$

in which:

y is a decimal number between 1.05 and 2;
S represents the remainder of a reducing sugar chosen from among glucose, xylose or arabinose.
$R_6$ represents a radical chosen from among the n-octyl, n-decyl, n-dodecyl, n-tetradecyl, n-hexadecyl radicals or a mix of compounds with formula (IV).

**11.** Process as defined in any one of claims 1 to 10, **characterised in that** the foaming surfactant to cross-linked anionic polyelectrolyte (P) ratio by mass is between 1/10 and 40/1.

**12.** Composition (C1) **characterised in that** it comprises the following for 100% of its mass:

- from 0.05% to 2% by mass of at least one anionic cross-linked polyelectrolyte (P) derived from polymerisation of at least one monomer with a strong acid function, said monomer being partially or completely salified 2-methyl 2-[(1-oxo 2-propenyl) amino] 1-propanesulfonic acid with at least one neutral monomer chosen from among N, N-dialkyl acrylamides, in which each of the alkyl groups comprises between one and four carbon atoms, and at least one monomer with formula (I) :

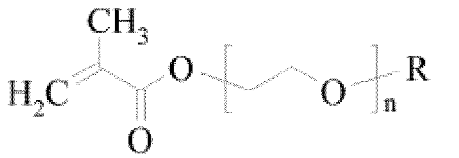

(I)

in which R represents a linear or ramified alkyl radical comprising from eight to twenty carbon atoms and n represents a number greater than or equal to 1 and less than or equal to twenty, in the presence of at least one cross-linking agent;
- from 10% to 50% by mass of at least one foaming surfactant selected from among anionic surfactants, non-ionic surfactants and amphoteric surfactants;
- from 0.01% to 10% by mass of at least one acid agent (A) selected from among partially or completely salified $\alpha$-hydroxy acids and $\beta$-hydroxy free acids;
- from 89.94% to 38% by mass of water;
and **in that** its pH is greater than or equal to 4.0 and is less than or equal to 6.5.

**13.** Composition (C1) as defined in claim 12, **characterised in that** the ratio of foaming surfactant to cross-linked anionic polyelecrolyte (P) by mass is between 1/10 and 40/1.

**14.** Composition (C1) as defined in either claim 12 or 13, **characterised in that** the acid agent (A) is selected from among elements in the group composed of lactic acid, citric acid, glycol acid, gluconic acid, tartric acid, malic acid and salicylic acid.

**15.** Use of a composition (C1) as defined in any one of claims 12 to 14, for cleaning and/or cleansing of face and/or body skin.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 200234793 A2
- WO 2011030044 A
- US 7025973 B2
- FR 2852257
- FR 2858554
- FR 2820316
- FR 2852258


### Littérature non-brevet citée dans la description


- **DANIEL VOET ; JUDITH G. VOET.** Biochemistry. John Wyley & Sons, 1990, 250